(19) **Europäisches Patentamt**

**European Patent Office**

**Office européen des brevets**

(11) Veröffentlichungsnummer : **0 296 328 B1**

(12) **EUROPÄISCHE PATENTSCHRIFT**

(45) Veröffentlichungstag der Patentschrift :
31.07.91 Patentblatt 91/31

(51) Int. Cl.⁵ : **C07C 409/26**

(21) Anmeldenummer : **88106179.0**

(22) Anmeldetag : **19.04.88**

(54) Verfahren zur Herstellung von Peressigsäure.

(30) Priorität : 22.06.87 DE 3720562

(43) Veröffentlichungstag der Anmeldung :
28.12.88 Patentblatt 88/52

(45) Bekanntmachung des Hinweises auf die
Patenterteilung :
31.07.91 Patentblatt 91/31

(84) Benannte Vertragsstaaten :
AT BE DE ES FR GB GR IT NL SE

(56) Entgegenhaltungen :
DE-B- 1 165 576
DE-B- 2 141 157

(73) Patentinhaber : Degussa Aktiengesellschaft
Weissfrauenstrasse 9
W-6000 Frankfurt am Main 1 (DE)

(72) Erfinder : Böhme, Georg
Nordring 49
W-6458 Rodenbach 1 (DE)
Erfinder : Hofen, Willi
Südring 54
W-6458 Rodenbach 1 (DE)
Erfinder : Prescher, Günter, Dr.
Liesingstrasse 2
W-6450 Hanau 9 (DE)
Erfinder : Siegmeier, Rainer, Dr.
Anspacherstrasse 35
W-6380 Bad Homburg (DE)

EP 0 296 328 B1

## Beschreibung

Eines der bekanntesten Oxydationsmittel ist Peressigsäure, vor allem zur Herstellung von Epoxiden oder von Lactonen aus Ketonen über die Baeyer-Villiger-Reaktion.

Peressigsäure wird im allgemeinen auf klassische Weise durch Umsetzen von Essigsäure mit Wasserstoffperoxid in Gegenwart eines sauren Katalysators, meist Schwefelsäure, hergestellt. Dabei fallen je nach der Art der Durchführung mehr oder weniger reine wäßrige Lösungen von Peressigsäure an, wie z.B. die sogenannte Gleichgewichtsperessigsäure, die z.B. aus ca. 40-42% Peressigsäure, 37-40% Essigsäure, 10-14% Wasser, 4-6% Wasserstoffperoxid und 0,5-1% Schwefelsäure besteht. (Ullmann's Enzyklopädie der technischen Chemie, 3. Auflage, Band 13, Seite 254).

Verwendet man ein Reaktionsgemisch, das außer Wasser und dem sauren Katalysator mehr als 1 Mol Wasserstoffperoxid pro Mol Essigsäure enthält, und destilliert die Peressigsäure unter vermindertem Druck mit Wasser ab, so erhält man sehr reine wäßrige Lösungen, siehe loc. cit., Ergänzungsband, 3. Auflage, Seite 181. Diese lassen sich durch azeotrope Schleppmitteldestillation in wasserfreie organische Peressigsäurelösungen überführen, siehe loc. cit. und DE-PS 1165576.

Auch durch die gleichzeitige Anwesenheit eines organischen — mit Wasser ein Azeotrop bildenden — Lösungsmittels bei der Umsetzung von Wasserstoffperoxid und Essigsäure — in Gegenwart eines sauren Katalysators — kann durch azeotrope Entfernung des Wassers eine wasserfreie, organische Lösung von Peressigsäure erhalten werden, siehe Ullmann, Ergänzungsband, loc. cit.

Bekannt sind aber auch die sicherheitstechnischen Risiken bei der Herstellung organischer Percarbonsäurelösungen. Aus diesem Grunde wurde versucht, durch direkte Extraktion wäßriger Mischungen von Percarbonsäure die entsprechenden organischen Lösungen zu erhalten, die durch Zusatz eines Schleppmittels azeotrop entwässert wurden. Geeignete Extraktionsmittel waren aliphatische, cycloaliphatische, aromatische Kohlenwasserstoffe, sowie chlorierte Kohlenwasserstoffe, siehe DE-OS 2145603. Wasserstoffperoxid wurde als 30 bis 90 gew.-%ige, bevorzugt als 45 bis 70 gew.-%ige, wäßrige Lösung im Überschuß über die Carbonsäure eingesetzt, und zwar bevorzugt in hochkonzentrierter wäßriger Form.

Auch Phosphorsäureester, bevorzugt Trialkylphosphate, wurden als Extraktionsmittel verwendet und die erhaltenen Extrakte mit Alkylestern niederer Carbonsäuren desorbiert. Die eingesetzten Percarbonsäuren waren 10-80, bevorzugt 20-60 gew.-%ige, wäßrige Lösungen, siehe US-PS 3,839,216.

Man sah ein Gefahrenmoment nur in der Tatsache, daß ein organisches Lösungsmittel neben Wasserstoffperoxid, Percarbonsäure und saurem Katalysator anwesend war und versuchte allein, diese Kombination zu vermeiden. Es zeigte sich aber, daß das Gefahrenmoment auch ohne Anwesenheit eines organischen Lösungsmittels auftrat.

Aus diesem Grunde wurde vor allem das Einsatzmolverhältnis von Wasserstoffperoxid zu Carbonsäure auf bestimmte Größen festgelegt, bevor die Umsetzung in Gang gebracht wurde. Es zeigte sich weiter, daß die Sicherheit des Systems nicht — wie angenommen — ab einer gewissen Größe kontinuierlich konstant blieb, sondern daß innerhalb der Bereiche dieses Einsatzmolverhältnisses eine ausgeprägte Sicherheitslücke auftrat, siehe DE-OS 2519299 und 2519300. Eine Rolle spielte dabei auch das Gewichtsverhältnis von Wasserstoffperoxid zu Wasser.

Aufgabe der Erfindung ist es daher, die Herstellung von Peressigsäure und ihre Gewinnung in einem organischen Lösungsmittel sicherheitstechnisch einwandfrei durchzuführen.

Es wurde nun gefunden, daß sich diese Aufgabe lösen läßt, wenn man die Umsetzung in einem einfachen Destillationsapparat derartig durchführt, daß man im Sumpf des Apparates vor Beginn der Umsetzung Wasserstoffperoxid und Essigsäure in einem Molverhältnis von 1 bis 2 : 1 und Wasserstoffperoxid in einer 30-35 gew.-%igen wäßrigen Lösung vorlegt, und daß man die Konzentration an Schwefelsäure auf 20-30 Gew.-%, bezogen auf die Gesamtlösung, einstellt, diesen Zustand der oben definierten Einsatzgrößen im Sumpf der Kolonne stationär aufrechterhält, die Reaktion bei Temperaturen von 55 bis 70°C und Systemdrücken von 100-200 mbar durchführt, aus dem Apparat eine Dampfphase aus Peressigsäure, Essigsäure und Wasser abnimmt, diese Dampfphase in eine Absorptionskolonne im Gegenstrom zu einem organischen Phosphat mit 3-30 Kohlenstoffatomen führt, das den Anteil an Peressigsäure und Essigsäure aufnimmt, wodurch man eine Lösung von Peressigsäure und Essigsäure in dem betreffenden Phosphat erhält, während der nicht absorbierte Wasserdampf über Kopf abgenommen wird und das System verläßt.

Unter einem "einfachen Destillierapparat" werden alle Apparate verstanden, die für eine einfache oder Geradeaus-Destillation bekannt sind, wie z.B. Blasen, Verdampfungskolben, Sumpfverdampfer, und die bevorzugt mit einem Kondensator oder Dephlegmator verbunden sind. Auch Kolonnen mit oder ohne Dephlegmator, die bevorzugt als Bodenkolonne ausgebildet sind, können eingesetzt werden. Als übliche Absorptionskolonnen kommen die in der Absorptionstechnik bekannten Apparate infrage. Das die Umsetzung und Absorption umfassende System wird bevorzugt bei Temperaturen von 45-65°C gefahren. Wird die Absorption z.B. bei 45-55°C gefahren, so ist es über das Absorptionsgleichgewicht möglich, eine 23 gew.-%ige Lösung von Peressigsäure in Tributylphosphat im Sumpf S II zu erhalten. Je höher die Absorptionstemperatur liegt, desto niedriger sind die Konzentrationen an Peressigsäure im Sumpf S II.

Der Reaktionsteil des Systems wird derartig betrieben, daß sich in der Dampfphase ein Gewichtsverhältnis von Peressigsäure zu Essigsäure von 2 bis 3 : 1, bevorzugt von 2,5 bis 3 : 1, einstellt, der Rest ist Wasser. Die bevorzugte Zusammensetzung des Dampfes entspricht 45-47 Gew.-% Peressigsäure, 14-17 Gew.-% Essigsäure, Rest Wasser.

Als organische Phosphate mit 3-30 Kohlenstoffatomen eignen sich trisubstituierte Phosphate, und zwar sowohl Alkyl-, Cycloalkyl- oder Arylphosphate, die auch als Mischungen eingesetzt werden können. Infrage kommen z.B. Trialkylester der Phosphorsäure mit 1-10 C-Atomen ; sowie Tricyclohexylphosphat oder Triphenylphosphat, Tricresylphosphat, Diphenylcresylphosphat. Bevorzugt sind Trimethyl-, Triäthyl-, Tributyl- und Trioctylphosphat.

Da aus dem System immer nur so viel Wasser abdestilliert wird, wie es dem Anteil aus der zugespeisten Wasserstoffperoxidlösung und dem Reaktionswasser entspricht, kann — unabhängig von der Verwendung einer 30-35 gew.-%igen wäßrigen Wasserstoffperoxidlösung als Vorlage — jede beliebige handelsübliche Lösung, z.B. 50-70%ig, für die kontinuierliche Umsetzung zugespeist werden.

Soll die Peressigsäure nicht als Lösung in einem Phosphorsäureester eingesetzt werden, so ist eine Überführung der Peressigsäure aus dem Phosphat in ein anderes organisches Lösungsmittel, wie z.B. aliphatische Ester, in üblicher Weise möglich. Diese Lösungsmittel müssen stabil gegen Oxydationen unter den üblichen Desorptionsbedingungen sein und sich leicht von Essigsäure destillativ trenran lassen. Bevorzugt sind Methyl- bis Propylacetate. Auch aromatische Kohlenwasserstoffe, z.B. Benzol, oder chlorierte Kohlenwasserstoffe, auch im Gemisch mit Estern, sind anwendbar.

Die Desorption der Peressigsäure und Essigsäure aus der Phosphatlösung kann in bekannter Weise durch Einführen des dampfförmigen zweiten Lösungsmittels vorgenommen werden.
Die entstehenden Desorbate können — falls gewünscht — durch Azeotropdestillation völlig entwässert werden.

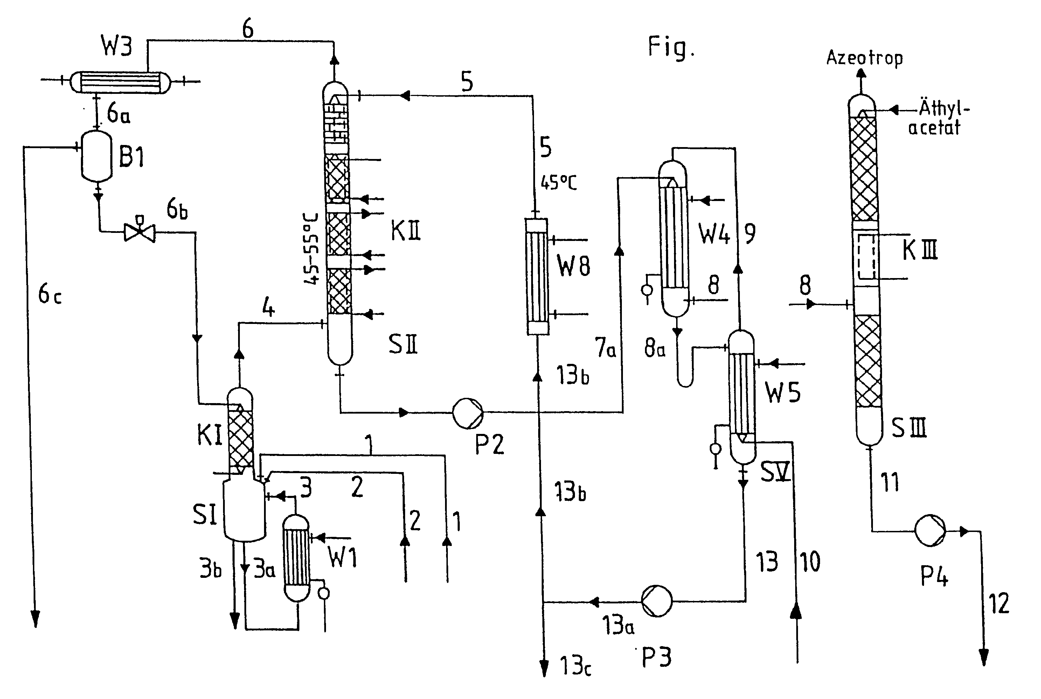

Das erfindungsgemäße Verfahren, das bevorzugt kontinuierlich durchgeführt wird, wird anhand der Abbildung näher erläutert. Die in der Beschreibung dieser Abbildung enthaltenen Zahlengrößen sind nur beispielhaft.

Als Destillationsapparat dient z.B. eine Bodenkolonne K I (mit aufgesetztem Dephlegmator). Im Sumpf S I der Kolonne werden — vor Beginn der Reaktion — Wasserstoffperoxid und Essigsäure (ES) im Molverhältnis 1,5 : 1 vorgelegt ; eingesetzt wird ein 40-50 gew.-%iges Wasserstoffperoxid. Durch Zufügen von Wasser wird das Gewichtsverhältnis von Wasserstoffperoxid zu Wasser auf 0,54 eingestellt (das entspricht einer 35 gew.-%igen Wasserstoffperoxidlösung), und Schwefelsäure wird in solcher Menge dem Gemisch zugegeben, daß deren Konzentration im Gemisch z.B. 25-30 Gew.-% beträgt.

Durch Anwenden eines Systemdruckes von 100-150 mbar, einem Rücklaufverhältnis von 0,3-0,5 und kontinuierlichem Zufügen von 40-50 gew.-%iger Wasserstoffperoxidlösung und Essigsäure im Zuspeiseverhältnis (Gewichtsverhältnis) von 0,7 bis 0,8 : 1 wird dieses vor Einsetzen der Umsetzung vorgelegte Sumpfgemisch während der Umsetzungsdauer so weit konstant gehalten, daß die auf den Einsatz vor Reaktionsbeginn berechneten Größen von Molquotient ($H_2O_2$/ES) und Gewichtsverhältnis ($H_2O_2$/$H_2O$) erhalten bleiben. Die Sumpftemperatur beträgt z.B. 60°C und wird mit Hilfe des Umlaufverdampfers W 1 über die Leitungen 3 und 3a aufrecht erhalten.

Unter diesen Umständen wird ein Brüdenstrom gewonnen, der aus ca. 47 Gew.-% Peressigsäure (PES), ca. 16 Gew.-% Essigsäure, ca. 37 Gew.-% Wasser und weniger als 0,1 Gew.-% Wasserstoffperoxid besteht, und über Leitung 4 in den unteren Teil (Sumpf S II) der Absorptionskolonne K II geführt wird. Auf den Kopf der Kolonne wird ein organisches Phosphat, z.B. Tributylphosphat (TBF) über Leitung 5 gegeben, das im Gegenstrom zu dem aufsteigenden Brüdenstrom geführt wird.

Das Phosphat absorbiert sowohl die Peressigsäure als auch Essigsäure aus dem Brüdenstrom, aber nur geringe Mengen an Wasser (sie entsprechen maximal der Löslichkeit von Wasser in Tributylphosphat). Nahezu das gesamte im Brüdenstrom vorhandene Wasser verläßt über Leitung 6 die Absorptionskolonne K II und wird im Kondensator W 3 kondensiert und gelangt über Leitung 6a in Abscheider B 1 und wird von dort über Leitung 6c ausgeschleust. Eine kleine Teilmenge wird als Rücklauf der Kolonne K I zugeführt, um Wasserstoffperoxid im Sumpf S I zurückzuhalten (über Leitung 6b).

Der Sumpf S II von Absorptionskolonne K II enthält — wie gesagt — die Peressigsäure und Essigsäure, absorbiert in Tributylphosphat. Es sollte stets so viel des Absorptionsmittels angewendet werden, daß bei einmaligem Durchgang des Brüdenstroms aus Destillationsapparat K I bei gegebener Temperatur und Druck praktisch keine Peressigsäure und Essigsäure mit dem Wasserdampf zusammen am Kopf von Kolonne K II entweichen. Dies läßt sich durch einen Vorversuch leicht ermitteln.

Im vorliegenden, beispielhaften Fall wurde mit Tributylphosphat bei einer Temperatur von 45-55°C und einem Druck von 100-150 mbar (da die Kolonnen K I und K II ein zusammenhängendes System bilden) eine 23 gew.-%ige Lösung von Peressigsäure neben 7 Gew.-% Essigsäure in Tributylphosphat erhalten. Die so erhaltene stabile Lösung von Peressigsäure in Tributylphosphat ist schon in dieser Form einsatzfähig. Sie wird aus Sumpf S II abgenommen (nicht gezeigt).

Falls jedoch Peressigsäure in einem anderen Lösungsmittel verwendet werden soll, z.B. in einem aliphatischen Ester, wie Äthylacetat, so wird die in Tributylphosphat erhaltene Lösung über Leitung 7, Pumpe 2 und Leitung 7a auf den Kopf des Verdampfersystems W 4 und W 5, das z.B. aus Fallfilmverdampfern besteht, geleitet und mit Hilfe von über Leitung 10 eingeführten Dämpfen von Äthylacetat, die über W 5 und Leitung 9 in W 4 eingeführt werden, desorbiert. Die Brüdenströme des Desorptionssystems gelangen über Leitung 8 in die Kolonne K III. Das von Peressigsäure und Essigsäure weitgehend befreite Tributylphosphat geht über Leitung 8a von W 4 nach W 5 und verläßt den Sumpf V über Leitung 13. Bevorzugt wird das aus S V ablaufende Tributylphosphat mittels Wasserdampfstrippung fast vollständig von gelöstem Äthylacetat befreit, (nicht gezeigt).

Der Brüdenstrom von Peressigsäure und Essigsäure in Äthylacetat wird — wie gesagt — im Sumpf von W 4 über Leitung 8 abgenommen und kann gegebenenfalls noch in Kolonne K III durch Bildung eines Azeotrops aus dem geringen Rest Wasser mit einem Teil des Äthylacetats, das über Kopf der Kolonne K III abgenommen wird, entwässert werden. Das bei der Entwässerung abdestillierte heteroazeotrope Gemisch aus Wasser und Äthylacetat wird in üblicher Weise kondensiert, Wasser ausgeschleust und Äthylacetat zurückgeführt (nicht gezeigt). Anderenfalls kann direkt kondensiert werden.

Aus dem Sumpf S III der Kolonne K III wird eine praktisch wasserfreie Lösung von Peressigsäure und Essigsäure in Äthylacetat über Leitung 11, Pumpe 4 und Leitung 12 abgenommen. Bei einem Kolonnendruck von 300 mbar wurde eine 23 gew.-%ige Lösung von Peressigsäure in Äthylacetat erhalten, die außerdem noch 7 Gew.-% Essigsäure enthielt. Es läßt sich also die gesamte, in Tributylphosphat vorliegende Menge an Peressigsäure in Äthylacetat überführen.

Je nach den Bedingungen des Systems "K I und K II" können die verschiedensten Konzentrationen an Peressigsäure in Trialkylphosphat bzw. einem anderen Lösungsmittel (durch Desorption der Trialkylphosphatlösung) erhalten werden.

Das weitgehend von Peressigsäure, d.h. im beispielhaften Fall bis auf 0,02 Gew.-% Peressigsäure, und von Essigsäure, d.h. bis auf 1,5 Gew.-% Essigsäure, befreite Tributylphosphat sammelt sich im Sumpf S V des Verdampfers W 5 an und wird über Leitung 13, Pumpe 3 und Leitung 13a und 13b in den Kühler W 8 zurückgeführt und dort auf die Absorptionstemperatur von z.B. 45°C abgekühlt. Danach gelangt das Tributylphosphat zurück auf den Kopf der Kolonne K II (über Leitung 5).

Über Leitung 13c wird ein Teilstrom an Tributylphosphat von Zeit zu Zeit ausgeschleust und gereinigt und so die Menge der sich ansammelnden Verunreinigungen auf einem niedrigen Niveau gehalten.

Um die Anreicherung von Verunreinigungen im Sumpf S I gering zu halten, muß über Leitung 3b eine geringe Ausschleusrate erzeugt werden, z.B. 2 Gew.-%, bezogen auf den Zulauf aus Wasserstoffperoxid, Wasser und Essigsäure, und kann nach Reinigung wieder in K I rückgeführt werden (nicht gezeigt).

Der technische Fortschritt des erfindungsgemäßen Verfahrens liegt einmal in der unbedingt sicheren Durchführung des Verfahrens, da die Wasserstoffperoxidkonzentration während der Herstellung der Peressigsäure im Sumpf des Herstellungsapparates von vornherein auf einem niedrigen Niveau liegt und bei der Umsetzung auf diesem Niveau gehalten wird. Damit wird das gefährliche Auftreten von Sicherheitslücken völlig vermieden. Diese Sicherheit wird noch gefördert durch die Aufarbeitung des wasserdampfhaltigen Brüdenstroms durch Absorption in einem organischen Lösungsmittel, wodurch das Entstehen einer kondensierten hochprozentigen wäßrigen Phase von Peressigsäure unmöglich wird.

Die nach dem erfindungsgemäßen Verfahren gewonnenen Lösungen von Peressigsäure in Alkylphosphaten sind selbst bei höheren Temperaturen auffallend stabil. So beträgt die Zersetzungsrate von einer Lösung, die 23 Gew.-% Peressigsäure neben 7 Gew.-% Essigsäure enthält, bei 55°C nur 0,5-0,8 Gew.-%/h in Tributylphosphat.

Ein weiterer Vorzug der erfindungsgemäß hergestellten Peressigsäurelösungen in Alkylphosphaten liegt in der geringen Verdampfungsgeschwindigkeit dieser Phosphate, so daß keine Aufkonzentrierung der Lösungen durch Verdampfen des Lösungsmittels erfolgen kann.

**Patentansprüche**

1. Verfahren zur Herstellung von Peressigsäure in organischer Lösung durch Umsetzung von Wasserstoffperoxid und Essigsäure in Gegenwart von Schwefelsäure und Überführung in ein organisches Lösungsmittel, dadurch gekennzeichnet, daß man die Umsetzung in einem einfachen Destillationsapparat derartig durchführt, daß man im Sumpf des Apparates vor Beginn der Umsetzung Wasserstoffperoxid und Essigsäure in einem Molverhältnis von 1 bis 2 : 1 und Wasserstoffperoxid in einer 30-35 gew.-%igen wäßrigen Lösung vorlegt, und daß man die Konzentration an Schwefelsäure auf 20-30 Gew.-%, bezogen auf die Gesamtlösung, einstellt, diesen Zustand der vorstehend definierten Einsatzgrößen im Sumpf der Kolonne durch Zuspeisen von wäßrigem Wasserstoffperoxid und Essigsäure stationär aufrecht erhält, die Reaktion bei Temperaturen von 55 bis 70°C und Systemdrücken von 100-200 mbar durchführt, aus dem Apparat eine Dampfphase aus Peressigsäure, Essigsäure und Wasser abnimmt, diese Dampfphase in eine Absorptionskolonne im Gegenstrom zu einem organischen Phosphat mit 3-30 Kohlenstoffatomen führt, das den Anteil an Peressigsäure und Essigsäure aufnimmt, wodurch man eine Lösung von Peressigsäure und Essigsäure in dem betreffenden Phosphat erhält, während der nicht absorbierte Wasserdampf über Kopf abgenommen wird und das System verläßt.

2. Verfahren nach Anspruch 1, dadurch gekennzeichnet, daß man als Destillationsapparat eine Bodenkolonne mit aufgesetztem Dephlegmator verwendet.

3. Verfahren nach Anspruch 1 und 2, dadurch gekennzeichnet, daß man vor Beginn der Reaktion Wasserstoffperoxid und Essigsäure im Molverhältnis 1,5 : 1 vorlegt, das Gewichtsverhältnis von Wasserstoffperoxid zu Wasser auf 0,54 und die Konzentration an Schwefelsäure im Gesamtgemisch auf 25-30 Gew.-% einstellt.

4. Verfahren nach Anspruch 1-3, dadurch gekennzeichnet, daß man die Reaktion bei einem Systemdruck von 100-150 mbar, einem Rücklaufverhältnis von 0,3 bis 0.5 und kontinuierlichem Zuspeisen von 40-50 gew.-%iger wäßriger Wasserstoffperoxidlösung und Essigsäure im Gewichtsverhältnis von 0,7 bis 0,8 : 1 durchführt.

5. Verfahren nach Anspruch 1-4, dadurch gekennzeichnet, daß man die Absorption der Dampfphase aus Peressigsäure, Essigsäure und Wasser in trisubstituierten Alkyl-, Cycloalkyl- oder Arylphosphaten durchführt.

6. Verfahren nach Anspruch 1-5, dadurch gekennzeichnet, daß man Trialkylphosphate mit 1-10 Kohlenstoffatomen einsetzt.

7. Verfahren nach Anspruch 1-6, dadurch gekennzeichnet, daß man Trimethyl-, Triäthyl-, Tributyl- oder Trioctylphosphat, bevorzugt Tributylphosphat, verwendet.

8. Verfahren nach Anspruch 1-7, dadurch gekennzeichnet, daß man die Lösung von Peressigsäure und Essigsäure in Tributylphosphat durch Desorbieren mit aliphatischen Estern, bevorzugt Äthylacetat, in eine Lösung des betreffenden Esters überführt.

## Claims

1. A process for the production of peracetic acid in organic solution by the reaction of hydrogen peroxide and acetic acid in the presence of sulphuric acid and transfer into an organic solvent, characterised in that the reaction is carried out in a simple distillation apparatus by introducing hydrogen peroxide and acetic acid in a molar ratio of from 1 : 1 to 2 : 1 and hydrogen peroxide as a 30-35% by weight aqueous solution into the sump of the apparatus before the beginning of the reaction and adjusting the concentration of sulphuric acid to 20-30% by weight, based on the whole solution, keeping this state of the above-defined starting magnitudes stationary in the sump of the column by the further addition of aqueous hydrogen peroxide and acetic acid, carrying out the reaction at temperatures from 55 to 70°C and system pressures of 100-200 mbar, removing a vapour phase of peracetic acid, acetic acid and water from the apparatus, and introducing this vapour phase into an absorption column in countercurrent to an organic phosphate having 3-30 carbon atoms which takes up the peracetic acid and acetic acid, whereby a solution of peracetic acid and acetic acid in the given phosphate is obtained while the unabsorbed steam is removed overhead and leaves the system.

2. A process according to Claim 1, characterised in that the distillation apparatus used is a plate column with dephlegmator attached.

3. A process according to Claims 1 and 2, characterised that hydrogen peroxide and acetic acid are introduced into the apparatus in a molar ratio of 1.5 : 1 before the beginning of the reaction, the ratio by weight of hydrogen peroxide to water is adjusted to 0.54 and the concentration of sulphuric acid in the whole mixture is adjusted to 25-30% by weight.

4. A process according to Claims 1-3, characterised in that the reaction is carried out at a system pressure of 100-150 mbar and a reflux ratio of 0.3 to 0.5 and with continuous infeed of 40-50% by weight aqueous hydrogen peroxide solution and acetic acid in a ratio by weight of 0.7 to 0.8 : 1.

5. A process according to Claims 1-4, characterised in that the absorption of the vapour phase of peracetic acid, acetic acid and water is carried out in trisubstituted alkyl-, cycloalkyl- or aryl-phosphates.

6. A process according to Claims 1-5, characterised in that trialkylphosphates having 1-10 carbon atoms are used.

7. A process according to Claims 1-6, characterised in that trimethyl-, triethyl-, tributyl- or trioctylphosphate is used, preferably tributylphosphate.

8. A process according to Claims 1-7, characterised in that the solution of peracetic acid and acetic acid in tributylphosphate is converted into a solution of the corresponding ester by desorption with aliphatic esters, preferably ethyl acetate.

## Revendications

1. Procédé de fabrication de l'acide peracétique en solution organique par décomposition du peroxyde d'hydrogène (eau oxygénée) et de l'acide acétique en présence d'acide sulfurique, et transfert dans un solvant organique, caractérisé en ce qu'on effectue la décomposition dans un simple dispositif de distillation dans lequel, on dispose au fond de l'appareil, avant le début de la réaction, du peroxyde d'hydrogène et de l'acide acétique dans un rapport de concentration molaire de 1 à 2 contre 1 et du peroxyde d'hydrogène en solution aqueuse à 30-35% en poids, et que l'on règle la concentration en acide sulfurique à 20-30% en poids, calculé sur la totalité de la solution, on maintient stationnaire l'état des paramètres définis ci-dessus dans le fond de la colonne au moyen d'une alimentation en peroxyde d'hydrogène aqueux et d'acide acétique, on effectue la réaction à une température comprise entre 55°C et 70°C et sous une pression de 100-200 mbar, on extrait de l'appareillage une phase vapeur constituée d'acide peracétique, d'acide acétique et d'eau, on transfère cette phase vapeur dans une colonne d'absorption parcourue à contre-courant par un phosphate organique avec 3 à 30 atomes de carbone, qui recueille la partie contenant l'acide peracétique et l'acide acétique et on obtient une solution d'acide peracétique et d'acide acétique dans le phosphate mentionné, tandis que la vapeur d'eau non absorbée est extraite au niveau de la tête (du dispositif) et quitte le système.

2. Procédé conforme à la revendication 1, caractérisé en ce qu'on utilise comme dispositif de distillation une colonne à plateau munie d'un déflegmateur.

3. Procédé conforme aux revendications 1 et 2, caractérisé en ce qu'avant le début de la réaction, on dispose le peroxyde d'hydrogène et l'acide acétique dans un rapport de concentration molaire de 1,5 contre 1, on règle le rapport de concentration pondérale du peroxyde d'hydrogène par rapport à l'eau à 0,54 et la concentration en acide sulfurique dans le mélange total à 25-30% en poids.

4. Procédé conforme aux revendications 1 à 3, caractérisé en ce qu'on effectue la réaction sous une pression dans le système de 100-150 mbar, avec un taux de recyclage de 0,3 à 0,5, et avec une alimentation continue d'une solution aqueuse de peroxyde d'hydrogène à 40-50% en poids et d'acide acétique, dans un rapport respectif de concentration pondérale de 0,7 à 0,8 contre 1.

5. Procédé conforme aux revendications 1 à 4, caractérisé en ce qu'on effectue l'absorption de la phase vapeur constituée d'acide peracétique, d'acide acétique et d'eau dans un dérivé trisubstitué alkyl phosphate, ou cyclo alkyl phosphate ou aryl phosphate.

6. Procédé conforme aux revendications 1 à 5, caractérisé en ce qu'on utilise des trialkyl phosphates avec 1 à 10 atomes de carbone.

7. Procédé conforme aux revendications 1 à 6, caractérisé en ce qu'on utilise le triméthyl phosphate, le triéthyl phosphate, le tributyl phosphate ou le trioctyl phosphate, préférentiellement le tributyl phosphate.

8. Procédé conforme aux revendications 1 à 7, caractérisé en ce qu'on transfère la solution d'acide peracétique et d'acide acétique dans le tributhyl phosphate par désorption avec des esters aliphatiques, de préférence de l'acétate d'éthyle, pour former une solution avec l'ester mentionné ci-dessus.

Fig.